(19) **Europäisches Patentamt / European Patent Office / Office européen des brevets**

(11) **EP 2 788 043 B1**

(12) # EUROPEAN PATENT SPECIFICATION

(45) Date of publication and mention of the grant of the patent:
**27.02.2019 Bulletin 2019/09**

(21) Application number: **12809433.1**

(22) Date of filing: **07.12.2012**

(51) Int Cl.:
*A61M 1/36* *(2006.01)*   *A61M 1/38* *(2006.01)*

(86) International application number:
**PCT/US2012/068412**

(87) International publication number:
**WO 2013/086298 (13.06.2013 Gazette 2013/24)**

(54) **METHODS AND APPARATUS FOR DONOR PLATELET PRE-COUNT IN CENTRIFUGAL BLOOD SEPARATOR**

VERFAHREN UND VORRICHTUNGZUR VORZÄHLUNG VON SPENDERTRHOMBOZYTEN BEI EINEM ZENTRIFUGALEN BLUTABSCHEIDER

PROCÉDÉS ET APPAREIL POUR LA NUMÉRATION PLAQUETTAIRE PRÉALABLE D'UN DONNEUR DANS UN SÉPARATEUR CENTRIFUGE DE SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2011 US 201161567855 P**

(43) Date of publication of application:
**15.10.2014 Bulletin 2014/42**

(60) Divisional application:
**18179628.5 / 3 398 627**

(73) Proprietor: **Terumo BCT, Inc.**
**Lakewood, CO 80215 (US)**

(72) Inventors:
• **FENDER, Logan**
  **Lakewood, Colorado 80226 (US)**
• **ZELINSKAYA, Alexandra**
  **Lakewood, Colorado 80027 (US)**

(74) Representative: **J A Kemp**
  **14 South Square**
  **Gray's Inn**
  **London WC1R 5JJ (GB)**

(56) References cited:
  **WO-A2-02/05059**    **US-A- 5 951 877**
  **US-A1- 2009 211 989**    **US-B1- 6 270 673**

**Description**

The present invention relates generally to the field of extracorporeal blood processing apparatus which is particularly useful in blood component collection, and more particularly, the present invention relates to apparatus for determining a donor's platelet level or "pre-count" in a centrifugal blood separator, preferably an aphaeresis system.

One well-known type of extracorporeal blood processing involves an aphaeresis system and procedure in which blood is removed from a donor or a patient (hereafter referred to as a donor), directed to a blood component separation device (e.g., centrifuge), and separated into various blood component types (e.g., red blood cells, white blood cells, platelets, plasma) for collection or therapeutic purposes. Some of these blood component types may either be collected or may be treated for therapeutic purposes and returned to a donor, while the remainder may simply be returned to the donor. Representative centrifugal blood processing systems are the TRIMA (trademark) and TRIMA ACCEL (trademark) aphaeresis machines available from CaridianBCT, Inc. Features of these systems are described in US Patents and patent applications including, for example, US Patents 7,052,606, and 6,773,413, and 6,200,287, and US Application 12/234,960.

A number of factors may affect the commercial viability of an aphaeresis system. One factor relates to the time and expertise required of an individual to prepare and operate the aphaeresis system. For instance, reducing the time required by the operator to complete an entire collection procedure, as well as reducing the complexity of these actions, can increase productivity or lower the potential for operator error. Moreover, reducing the dependency of the system on the operator may further lead to reductions in the credentials desired/required for the operators of these systems. Characteristics of the fluids during the collection process may be sensed by various sensors in order to automate the separation process, as far as possible. An exemplary sensor illuminates a tube transporting fluid and detects ratios of reflected or transmitted red and green light from the fluid. The presence of red blood cells can be detected. Such a sensor is described in US Patent 5,734,464. Nevertheless, accurate and consistent control of a high-speed centrifugal blood separation system is difficult and complex, and further improvement in the control of possible failure modes is desirable.

US 2009/0211989 discloses a method for estimating the platelet pre-count of a donor when the platelet yield is known. This method is based on comprising measured values to stored empirical values for different weights, hematocrits and gender of donors.

The invention is defined in the claims.

The present invention generally relates to extracorporeal blood processing, and in particular, to improved sensing and control during priming of a blood processing machine. "Priming" refers to the process of preparing a blood processing machine for the separation process and includes loading the machine with fluid. The initializing fluid may be saline solution or some other fluid. In certain machines, such as the Trima Accel machine, the donor's blood may be used for the priming procedure. Since each of the various aspects of the present invention may preferably be incorporated into an aphaeresis system (e.g., whether for blood component collection in which "healthy" cells or other blood components are removed from the donor blood for later transfusion, or for therapeutic "unhealthy" blood component removal), the present invention will be described in relation to such aphaeresis systems. Aphaeresis may often imply the return of certain blood components back to the donor. However, certain aspects of the present invention may be suited for extracorporeal blood processing applications in which all donated blood components are retained and such are also intended within the scope of the present invention.

An aphaeresis system which may be used with one or more aspects of the present invention generally includes at least a blood component separation device, which provides the mechanism and/or the forces required to separate blood into various blood component types, such as red blood cells, white blood cells, platelets, or plasma. In one preferred embodiment, the separation device includes a centrifuge channel which receives a disposable blood processing vessel. Typically, a donor or perhaps a patient (collectively referred to hereafter as a donor) is fluidly interconnected with the blood processing vessel by an extracorporeal tubing circuit, and preferably the blood processing vessel and extracorporeal tubing circuit collectively define a closed, sterile system. When the fluid interconnection is established, blood may be extracted from the donor and directed to the blood component separation device such that at least one type of blood component may be separated and removed from the blood, either for collection or for therapy. An additive/storage solution is added to the red blood cells or platelets after collection. A blood return reservoir has heretofore been used to receive selected blood components before returning those components to the donor.

In certain procedures, platelets are collected from the donor. It is advantageous to predict the length of time that will be needed for the donation of a selected amount of platelets. Usually, this calculation is performed using a typical platelet concentration or the donor's platelet count, if it is known. The donor's pre-count may be known from a separate, pre-donation test, or from the donor's past donations. A pre-count test is usually performed on a separate machine and entails a delay in starting the donation procedure. The donor's platelet count history from past donations must be derived from written or electronic records, which may not be available. It is desirable, therefore, to provide an automated test for determining the donor's platelet pre-count, which test can be integrated into the apheresis machine itself. The information derived from the test may then be used in the prediction of platelet yield and in the platelet collection process.

The present invention uses a Red Blood Cell Detector (RBC Detector), incorporated into an apheresis machine such as the TRIMA ACCEL ® apheresis machine, in a new way to determine the donor's platelet count. The invention causes the apheresis machine to detect the initial exit of platelets from a leukoreduction chamber and to determine the donor platelet pre-count from elapsed time and the pre-determined volume of certain portions of a disposable bag set mounted on the apheresis machine. Certain conditions must be met for a valid measurement of the donor's platelet count to be made.

The system may also be used to determine a donor's hematocrit by detecting an initial exit of red blood cells.

These and still further aspects of the present invention are more particularly described in the following description of the preferred embodiments presented in conjunction with the attached drawings which are described briefly below.

Fig. 1 is a schematic view of an aphaeresis system.
Fig. 2 illustrates a tubing and bag set including an extracorporeal tubing circuit, a cassette assembly, and collection bag assembly for use with the system of Fig. 1.
Fig. 3 shows a red light-green-light RBC detector for use in the aphaeresis system.
Fig. 4 shows a second configuration of the RBC detector of Fig. 3.
Fig. 5 (comprising Fig. 5A, Fig. 5B, and Fig. 5C) shows a flow chart of a software implementation of a method.

The present invention will be described in relation to the accompanying drawings. Generally, the present invention relates to improvements for a blood processing aphaeresis system.

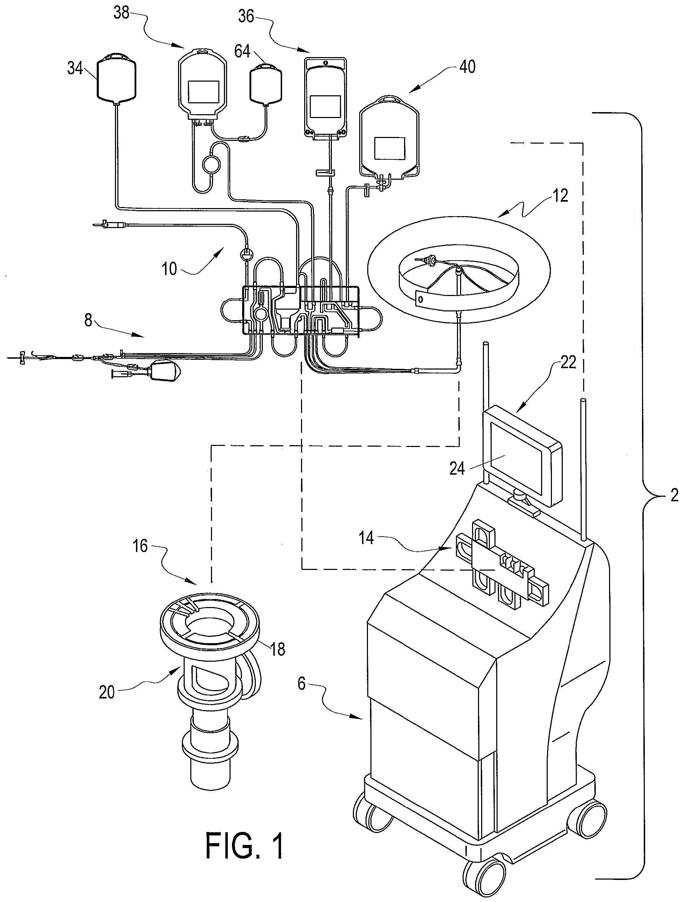

A preferred blood aphaeresis system 2 for use in and/or with the present invention is schematically illustrated in Fig. 1. System 2 preferably provides for a continuous blood component separation process. Generally, whole blood is withdrawn from a donor and is substantially continuously provided to a blood component separation device 6 where the blood is continuously separated into various component types and at least one of these blood component types is collected from the device 6. One or more of the separated blood components may then either be provided for collection and subsequent use by another through transfusion or may be uncollected and then returned to the donor. Therapeutic treatment and near immediate return of certain separated blood components is a viable, yet less common alternative use as well. It is also understood that for therapeutic treatment the blood may be separated into components with filtration using the principles of the instant invention and as described below at a patient's bedside for return to such patient.

In the blood aphaeresis system 2, blood is withdrawn from the donor and directed through a pre-connected bag and tubing set 8 which includes an extracorporeal tubing circuit 10 and, in one embodiment, a blood processing vessel 12 which together define a closed, sterile and disposable system. The set 8 is adapted to be mounted on the blood component separation device 6. The separation device 6 preferably includes a pump/valve/sensor assembly 14 for interfacing with the extracorporeal tubing circuit 10, and a channel assembly 16 for interfacing with the disposable blood processing vessel 12.

The channel assembly 16 may include a channel housing 18 that is rotatably interconnected with a rotatable centrifuge rotor assembly 20, which provides the centrifugal forces required to separate blood into its various blood component types by centrifugation. The blood processing vessel 12 may be fitted within the channel housing 18. When connected as described, blood can be flowed substantially continuously from the donor, through the extracorporeal tubing circuit 10, and into the rotating blood processing vessel 12. The blood within the blood processing vessel 12 may then be continuously separated into various blood component types and at least one of these blood component types (platelets, plasma, or red blood cells) may be removed from the blood processing vessel 12. Blood components which are not being retained for collection or for therapeutic treatment are preferably also removed from the blood processing vessel 12 and returned to the donor via the extracorporeal tubing circuit 10. Various alternative aphaeresis systems (not shown) may also make use of the present invention, including batch processing systems (non-continuous inflow of whole blood or non-continuous outflow of separated blood components) or smaller scale batch or continuous RBC/plasma separation systems, whether or even if no blood components may be returned to the donor.

Operation of the blood component separation device 6 is preferably controlled by one or more processors included therein, and may advantageously comprise a plurality of embedded computer processors to accommodate interface with ever-increasing PC user facilities (e.g., CD ROM, modem, audio, networking and other capabilities). In order to assist the operator of the aphaeresis system 2 with various aspects of its operation, the blood component separation device 6 may include a graphical interface 22 with an interactive touch screen 24.

Further details concerning the operation of an aphaeresis system, such as the TRIMA (trademark) System and the TRIMA ACCEL (trademark) System (available from the assignee of this application, CaridianBCT, Inc., of Lakewood, Colorado) may be found in a plurality of publications, including, for example, US Patents 5,734,494, US 7,052,606, US 6,773,413, and US 6,200,287, and US Applications 12/234,960 and 12/423,883. A plurality of other known aphaeresis systems may also be useful herewith, as for example, the Baxter CS3000, AMICUS, AUTOPHERESIS-C, and ALYX systems or the Haemonetics MCS and MCS+, or the Fresenius COM.TEC and AS-104 or like systems.

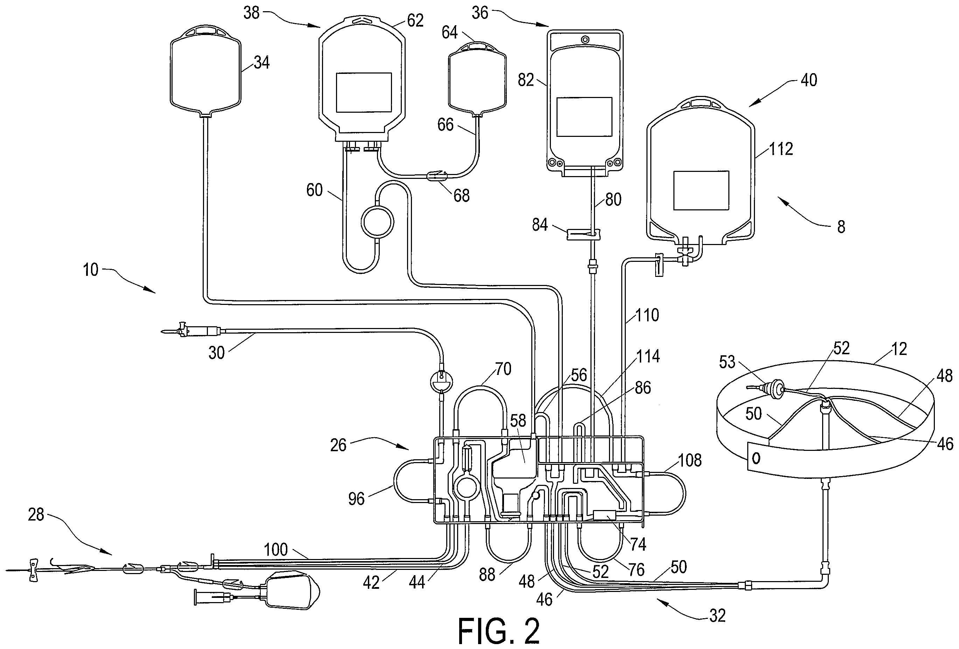

As illustrated in Fig. 2, the pre-connected extracorporeal tubing circuit 10 is shown which may include a cassette assembly 26 and a number of tubing/collection assemblies 28, 30, 32, 34, 36, 38 and 40 interconnected therewith. A blood removal/return tubing assembly 28 provides a single needle interface between a donor and the remainder of the tubing circuit 10 (although a two-needle set-up, not shown, may also be used). At least two lines 42, 44 are provided in assembly 28 for removal of blood from and return of components to the donor. This embodiment includes a cassette assembly 26, which is connected between the blood removal/return tubing assembly 28, and a blood inlet/outlet tubing assembly 32, which provides the coupling between cassette assembly 26 and blood processing vessel 12. Four lines 46, 48, 50 and 52 are shown in Fig. 2 for transport of blood, blood components and other fluids to and from the processing vessel 12. An anticoagulant tubing assembly 30, a plasma collection assembly 36, a red blood cell collection assembly 38, a vent bag tubing line assembly 34, and a platelet assembly 40 are also interconnected with cassette assembly 26 in this embodiment. The extracorporeal tubing circuit 10 and blood processing vessel 12 are preferably pre-connected as a closed, pre-sterilized, disposable assembly for a single use.

An RBC (red blood cell) outlet tubing line 48 of the blood inlet/blood component tubing assembly 32 connects the processing vessel 12 with an RBC return tubing loop 56 to return separated RBCs to a donor. For such purpose, the RBC return tubing loop 56 is preferably connected to the top of a blood return reservoir 58 of the cassette assembly 26. The tubing line 48 may also be connected with an RBC collection tubing assembly 38 for collecting RBCs. RBC collection tubing and bag assembly 38 includes an RBC collector tubing line 60, an RBC collection reservoir or bag 62, and an air removal bag 64. The air removal bag 64 is attached to the RBC collection bag 62 by a tubing line 66 which may have an optional clamp 68 attached thereto.

Plasma tubing 50 of blood inlet/blood component tubing assembly 32 connects through an integral plasma passageway to a pump-engaging, plasma tubing loop 76.

Through an integral plasma passageway, the plasma tubing loop 76 connects to the plasma collection tubing assembly 36 via tubing line 80. The plasma collection tubing assembly 36 may be employed to collect plasma during use and includes plasma collector tubing 80 and plasma collection bag 82. A slide clamp 84 may be provided on plasma collector tubing 80. The plasma tubing loop 76 is also connected to a plasma return tubing loop 86 to return plasma to donor/patient. For such purpose, the plasma return tubing loop 86 is connected through loops 108 and 114 to the top of the blood return reservoir 58 of the cassette assembly 26.

Platelet collect tubing 52 passes through a leukoreduction or LRS chamber 53. Platelets collect in the LRS chamber, forming a fluidized bed in consequence of the balance fluid flow, centrifugal forces and the size and weight of the platelets. When the fluidized bed of platelets within the LRS chamber becomes saturated or full, platelets begin to spill out of the LRS chamber into the platelet collect tubing 52, as will be explained further below in connection with the present method. The platelet collect tubing 52 connects through an integral passageway past a red/green light sensor 74, described below, to a pump-engaging loop 108 and either to a return loop114 connected to the reservoir 58 or to the platelet collection assembly 40. The platelet collection assembly 40 comprises a connecting tube 110 and one or more bags 112. A valve (not shown) in the cassette directs the collected platelets either to the return loop 114 or to the platelet collection assembly.

One or more types of uncollected blood components, e.g., red blood cells, plasma, or platelets (collectively referred to as return blood components) will cyclically accumulate in and be removed from reservoir 58 during use. Here also, valve/clamp access is made through cassette assembly 26 to maintain the plasma collector tubing 80 and plasma return tubing loop 86 in a predetermined spaced relationship for flow control therethrough.

Most portions of the tubing assemblies 28, 30, 32, 36, 34, 38, and 40 and cassette assembly 26 are preferably made from plastic components including, for example, polyvinyl chloride (PVC) tubing lines, that may permit visual observation and monitoring of blood/blood components during use. It should be noted that thin-walled PVC tubing may be employed for approved, sterile docking (i.e., the direct connection of two pieces of tubing line) for the RBC collector tubing lines 60, inter alia. All tubing lines are pre-connected before sterilization of the total disposable assembly to assure that maximum sterility of the system is maintained. A highly desirable advantage of pre-connection of all of the elements of the tubing circuit including the collection bag sub-assembly 38 involves the complete pre-assembly and then sterilization hereof after pre-assembly such that no sterile docking is later necessary (spike addition of storage solution excepted). Thus, the costs and risks of sterile docking are eliminated. Alternatively, thicker-walled PVC tubing may be employed for approved, sterile docking RBC collector tubing lines 60, inter alia.

As mentioned, a cassette assembly 26, may be mounted upon and operatively interface with the pump/valve/sensor assembly 14 of a blood component separation device 6 during use. Further details of an aphaeresis system set-up including the loading and interaction of a disposable assembly 8 with a blood component separation device 6, may be found in the above-listed patents, inter alia, and are not exhaustively repeated here.

Except as expressly set forth herein, operations of the aphaeresis process are preferably carried out as set forth in the above-listed patents. During a blood removal, whole blood will be passed from a donor into tubing line 44 of blood removal/return tubing assembly 28 and is then transferred to blood component separation device 6. At device 6, the blood is pumped via loop 88, to the processing vessel 12 via the cassette assembly 26 and inlet line 46 of the blood inlet/blood component tubing assembly 32. Separation processing then occurs on a substantially continuous basis in vessel 12; i.e., blood flows therein, is separated and flows as separated components therefrom. After separation processing in vessel 12 (though separation is continuously occurring), uncollected blood components are transferred from the processing vessel 12 to and through cassette assembly 26, into and may then accumulate in reservoir 58 of cassette 26 up to a predetermined level at which the blood component separation device 6, in a single needle operation, may (though in a continuous system, need not) pause the blood removal and initiate a blood return wherein these uncollected and/or treated components may be returned to the donor. As such, these accumulated components may be transferred through a pump-engaging loop 70 into the blood return tubing line 44 of blood removal/return tubing assembly 28 and back into the donor. During the single needle blood return, when the accumulated return blood components in reservoir 58 are removed down to a predetermined level, blood component separation device 6 will then automatically end the blood return. This preferably will also automatically serve to reinitiate or continue the blood removal. The cycle between blood removal and blood return will then continue until a predetermined amount of collected blood components have been harvested. In an alternative dual needle scheme, as is known in the art, blood may be continually removed from and blood components continually returned to a donor. The detailed mechanisms for such operations, including controlling the pumps, for example, are not shown or described in detail herein.

Also, certain components may be collected simultaneously or consecutively one after the other. In one example, platelets may be collected prior to collection of RBCs while plasma may be collected concurrently with either. In the primary example shown, three components are collected: RBCs in the RBC sub-assembly 38 and plasma in assembly 36 and platelets in the other collection assembly 40. When a sufficient quantity of one or the other is collected, further separated portions of such a component are returned to the donor with any other uncollected components, until a sufficient quantity of all components are collected. One or two selected components may be collected with all other components being returned to the donor.

In normal operation, whole blood will pass from the donor through the needle and blood removal tubing assembly 28, cassette assembly 26 and blood inlet tubing line 46 to processing vessel 12. The whole blood will then be separated in vessel 12. Also, a platelet stream or a plasma stream may be separated herein and be either collected in a collector assembly 40 or 36, or diverted to reservoir 58 for ultimate return to the donor. Separated plasma may be flowed through cassette 26 via loop 76 and line 86 for collection in the container 82 for plasma or diverted to reservoir 58. Separated platelets may be flowed through cassette 26 past red/green light sensor 74 via loop 108 and line 110 for collection in the container 112 or diverted to reservoir 58 through loop 114. Further, red blood cells (including potentially some white blood cells) may be separated in and passed from vessel 12 through RBC collect line 48, through cassette assembly 26 and, in return mode, into reservoir 58. In a preferred alternative, during an RBC collection procedure separated RBCs will be delivered to RBC collector tubing and bag assembly 38 through tubing line 60 for collection.

One preferred protocol, which may be followed for performing an aphaeresis procedure relative to a donor utilizing the described system 2, will now be summarized. Initially, an operator loads the disposable plastic assembly 8 in and/or onto the blood component separation device 6. According hereto, the operator hangs the various bags on hooks on the blood component separation device 6. If one is used, the operator then also loads the cassette assembly 26 on the device 6 and/or the blood processing vessel 12 within the channel housing 18 as mounted on the centrifuge rotor assembly 20 in the machine 6.

With the extracorporeal tubing circuit 10 and the blood processing vessel 12 loaded in the described manner, the donor may then be fluidly connected with the extracorporeal tubing circuit 10 by inserting an access needle of the needle/tubing assembly 28 into the donor. In addition, the anticoagulant tubing assembly 30 is primed by passing anticoagulant solution from a bag (not shown through a pump-engaging loop 96 to a line 100, which adds controlled amounts of anticoagulant to the blood near the needle. The blood removal/return tubing assembly 28 is primed preferably with blood from the donor. The blood processing vessel 12 is also primed for the aphaeresis procedure. A blood prime may be used in that blood will be the first liquid introduced into the blood processing vessel 12. During the priming procedure, as well as throughout the remainder of the aphaeresis procedure, blood may be flowed into the vessel 12, blood components are separated from each other and one or more components is removed from the blood processing vessel 12. Further details on a general blood processing procedure are set forth in US Application 12/234,940.

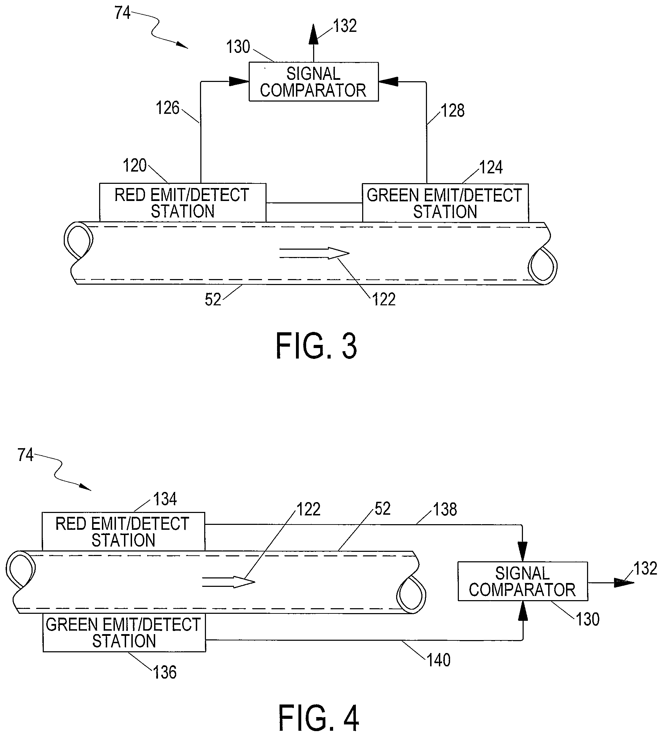

As shown in Figures 1 and 2, aphaeresis system 2 includes four relatively transparent tubes: blood component inlet tube 46 and collect tubes 48, 50 and 52. Collect tube 48 is intended to carry red blood cells. Plasma collect tube 50 carries plasma. Platelet collect tube 52 is intended to carry platelets, with the flow rate of the blood component within collect tube 52 typically in the range of from about 0.8 to about 25 ml/min. In Fig. 3, the direction of blood component flow is from left to right, as is shown by the arrows associated with platelet collect tube 52. In this embodiment, tubes 48, 46, 50 and 52 are constructed of optically transparent polyvinylchloride and are generally circular in cross section, and having an inner diameter of about 2.87 mm and an outer diameter of about 4.75 mm.

A red blood cell spillover detector 74 useful in the present invention is associated with platelet collect tube 52. The detector 74 includes light sources and mating light detector(s). In accordance with an important feature of the invention, both light sources and the mating light detector(s) that are within detector 74 are located on the same side of platelet collect tube 52.

Red light and green light are directed toward the blood collect tube that is to be monitored to determine the donor platelet pre-count. As used herein, the term green light is intended to mean visible electromagnetic radiation having a wavelength of from about 4,912 to about 5,750 angstroms, and the term red light is intended to mean visible electromagnetic radiation having a wavelength of from about 6,470 to about 7,000 angstroms.

Detection of red and green light can occur during the same time interval, in which case two light sensors or light detectors are provided, one sensor being selectively responsive only to red light reflection and the other sensor being selectively responsive only to green light reflection. However, in a preferred embodiment, the two tests occur during two different but closely spaced time intervals. In this embodiment, only one sensor may be provided, this one sensor having a wide wavelength response so that it is responsive to red light reflection during one time interval, and is responsive to green light reflection during another time interval.

The magnitude of the red light reflection and the magnitude of the green light reflection are compared. In a preferred embodiment, the ratio of red light reflection magnitude to green light reflection magnitude is determined. A compare function that compares the ratio to a user supplied threshold value, which is the minimum spillover tolerated or lacking in adverse consequences for a given application.

FIG. 3 shows an embodiment of the detector 74 wherein platelet collect tube 52 has associated therewith a first emit/detect station 120 in accordance with the invention that operates to emit a first color of light (red) into collect tube 52, and then to detect the reflection of this first color from the blood component flow 122 within collect tube 52. Spaced a short distance from station 120 is a second emit/detect station 124 that operates to emit a second color of light (green) into collect tube 52, and then to detect the reflection of this second color from the blood component flow 122 within collect tube 52.

The two respective output conductors 126 and 128 of stations 120 and 124 carry electrical signals whose magnitudes are directly proportional to the magnitude of the reflected first light and to the magnitude of the reflected second light. An electrical signal comparator 130 operates to compare the two signals on conductors 126, 128, and to provide an output on output conductor 132 as a result of this comparison.

The two stations 120,124 may operate during the same time interval having duration of, for example, fractions of a second. In this case the two signals on conductors 126 and 128 also appear during this common time interval. Signal comparator 130 may or may not include a latch means (not shown) that saves the magnitudes of these two signals to enable a ratio calculation that takes advantage of the fact that red light reflection increases and green light reflection decreases as the concentration of red blood cells increases.

The two stations 120, 124 may also operate during two different time intervals that are spaced from one another. In this case, the two signals on conductors 126 and 128 also appear during these two different time intervals, and signal comparator includes a latch means (not shown) that saves the magnitudes of these two signals in order to enable the ratio calculation to be made after expiration of the later or second of the two time intervals.

While the physical spacing of the two stations 120, 124 along the length of collect tube 52 is not critical to the invention, it may be desirable to maintain this spacing to a minimum, and/or to time the later operation of station 124 relative to the earlier operation of station 120, as a function of the flow rate of the blood component within collect tube 52. In this way, both station 120 and station 124 operate on the same flowing volume of blood component 122.

Fig. 4 shows an embodiment wherein platelet collect tube 52 has associated therewith a first emit/detect station 134 that emits a first color of light (red) into collect tube 52, and then detects the reflection of this first color from the blood component flow 122 within collect tube 52. Located diametrically across from station 134 is a second emit/detect station 136 that emits a second color of light (green) into collect tube 52, and then detects the reflection of this second color from the blood component flow 122 within collect tube 52.

Two respective output conductors 138 and 140 of stations 134 and 136 carry electrical signals whose magnitudes are directly proportional to the magnitude of the reflected first light and to the magnitude of the reflected second light. The electrical signal comparator 130 compares the two signals on conductors 138, 140, and provides an output on output conductor 132 as a result of this comparison.

The two stations 134, 136 may operate during the same time interval, whereupon the two stations 134,136 include individual light detectors that are selectively responsive only to the first light for the detector of station 134, and to the second light for the detector of station 136. In this case, the two signals on conductors 138 and 140 also appear during this time common interval. Comparator 130 may or may not include a latch means (now shown) to save the magnitudes of these two signals as the signal comparison is made. The two stations 134, 136 may also operate during two different time intervals. In this case, the light detector within each of the two stations 134, 136 is rendered operative only during the period of operation of that respective station 134, 136. Since interference is precluded by providing different time periods of operation for the two light detectors, the two light detectors may be of a relatively wide color response. The two electrical signals on conductors 138 and 140 also appear during these two different time intervals, and signal comparator 130 in this case includes a latch means (not shown) that operates to latch the magnitudes of these two signals 138, 140 in order to enable a comparison to be made after expiration of the later or second time interval.

The signal comparison provided at 130 is a ratio calculation, which takes advantage of the fact that red light reflection increases and green light reflection decreases as the concentration of red blood cells increases.

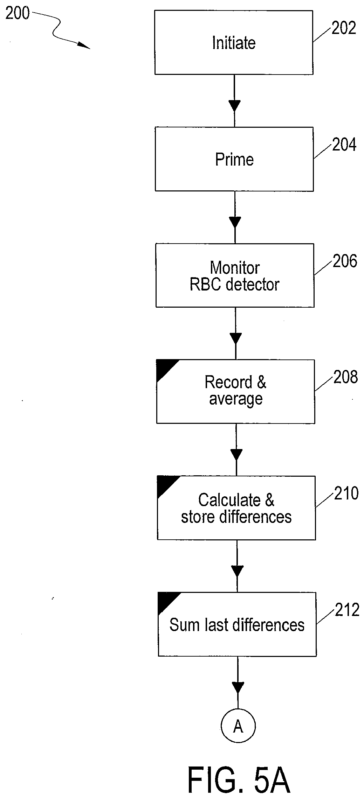

This invention uses the detected red-green ratio in a new way to determine a donor platelet pre-count. In the present invention, a collection valve controlling access to the platelet collect bag or assembly 40 does not open until platelets exit the LRS chamber 53 through platelet collect line 52. A method for validly determining a platelet pre-count will now be described in connection with a flow chart 200 of a program illustrated in FIG. 5A-C.

The apheresis machine 6 begins with a cell separation and collection process with an initial prediction 202, derived from an average platelet pre-count value for the characteristics (height, weight, sex) for the donor. However, the program prevents platelet collection from starting before a pre-determined quantity of blood (for example, 1000 mL) has passed through the separation bag 12, to allow for a pre-count measurement to be made as described below. The apparatus begins to prime 204 the extracorporeal tubing circuit 10 in a known manner. After a small amount of fluid (for example, 150 mL) has been introduced into the separation chamber 12, the apparatus begins to monitor 206 the output of the RBC detector 74. A relatively clear fluid (saline or plasma) is initially passing the RBC detector as platelets accumulate in the LRS chamber. The object of the process described herein is to detect the initial spill-over of slightly more opaque platelets out of the LRS chamber and to calculate the donor pre-count from the volumes of those portions of the extracorporeal tubing circuit 10 that contain separated platelets.

At a pre-determined sampling rate, for example one measurement every 0.5 seconds, the apparatus records 208 the red light reflectance signals for a volume of blood processed (VBP). In particular, the value stored for a particular VBP may be an average of the latest reflectance measurements, for example, the average of the last six measurements, which will be referred to as the "VPB average reflectance". The system also calculates the difference 210 between the current VPB average reflectance and the immediately preceding VPB average reflectance, and stores this series of values. These values will be referred to as the "average reflectance difference". Finally, the apparatus calculates and stores the sum 212 of a pre-determined number of average reflectance differences, for example, the last five average reflectance differences, each of which will be referred to as an "average reflectance sum". As is apparent, the apparatus thus associates three representations of the stability of the red light reflectance signal with a particular volume of blood processed. If these three representations meet certain criteria, described below, the apparatus can reliably detect the initial overflow of platelets out of the LRS chamber and can use the detected volume of blood processed to calculate the donor's platelet pre-count.

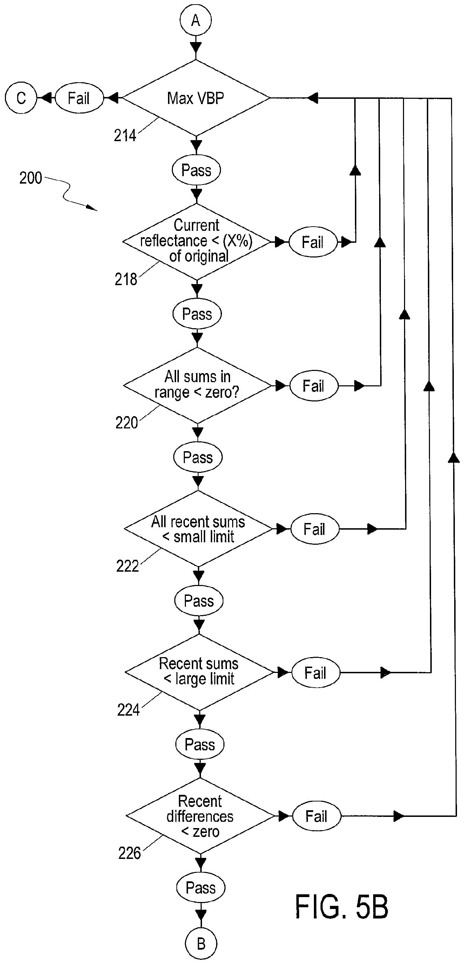

As shown in FIG. 5B, therefore, the apparatus test for a maximum allowable VBP 214. If the volume of blood processed exceeds the pre-determined maximum allowable VBP, for example 1450 mL, it is assumed that the LRS chamber has filled, but that the initial spill-over of platelets was not detected reliably. Consequently, the apparatus proceeds with processing 216 using the initial estimated pre-count value derived, as explained above, from the donor's characteristics. If the maximum allowable VBP has not yet been reached, the apparatus applies a series of tests for detection of the initial spill-over of platelets. Because the reflectance of platelets is much closer to the reflectivity of plasma than the reflectivity of red blood cells, platelets are much harder to detect than red blood cells. Preferably, each of the following five criteria should be met to detect a platelet spill-over. Criteria 1 (step 218): the current average reflectance must be at least less than a pre-determined part (for example, 85%) of the initial value for average reflectance. Criterion 1 implies that there has been a significant change in the reflectivity of the fluid passing through the detector. Criterion 2 (step 220) examines a set of average reflectance sums for a range of VBP prior to the current measurements. For example, the apparatus may examine the eighth through seventeenth measurements counting backwards from the current measurements. If each of the ten average reflectance sums for these ten VBP instances is less than zero, criterion 2 has been met. Meeting these criteria implies that there has been a relatively continuous decrease in reflectivity in the preceding time period. In the present example, this would be about 4 to 8 seconds prior to the current measurement. Criterion 3 (step 222) examines the average reflectance sums for the period from about 8 seconds to ten seconds prior to the current measurements. For example, if the eighteenth through twenty-first average reflectance sums prior to the current measurements were each less than -10, criterion 3 have been met. This is more stringent than criterion 2 and implies a more rapid change in reflectivity. Similarly, criterion 4 (step 224) is even more stringent. If for twenty-two (about ten to eleven seconds) immediately preceding average reflectance sums, the sum of all of these sums is less than -15, criterion 4 has been met. Finally, criterion 5 (step 226) requires that each of the average reflectance differences from about 4 to ten seconds prior to the current measurement (that is, the eighth through twenty-first VBP prior to the current measurement) each be less than 0.

Failure to pass each criterion returns the program to the maximum allowable VBP test 214. If the time limit set in that test has not expired, the five criteria will be tested serially. Thus, the first criterion 218 will usually be tested more frequently than the second criterion 220, since failure of data to meet the first criterion returns the program directly to the maximum allowable VBP test 214. Similarly, the second criterion 220 will usually be tested more frequently than the third criterion 222, and so on through the fifth criterion 226. If all criteria are met, a VBP can be confirmed (step 228) for collection of an initial volume of platelets (hereafter, "confirmed VBP") and the collect valve to the platelet bag or assembly 40 can be opened. Preferably, the confirmed VBP precedes the currently measured VBP, more preferably, the confirmed VBP is the twenty-second measurement preceding the current measurement, that is, the VBP corresponding to measurements taken about ten to eleven seconds prior to the current measurements. This corresponds to the beginning of the period in which the five criteria are met. Thus, it is believed that the initial spill-over of platelets out of the LRS chamber can be reliably identified from the characteristics of the fluid flow immediately after the initial spill over, rather than from the initial spill-over itself.

With the confirmed VBP established, the apparatus can calculate (step 230) the donor's platelet pre-count. The average size of platelets is known. The volume of the LRS chamber and of the segment of tubing or channel between the LRS chamber 53 on the rotor 18 and the RBC sensor 74 on the device 6 is known. The concentration of platelets in a fluidized bed under selected conditions of flow speed and centrifugal force can be calculated or derived from experiments.

$$PCT = \frac{(NPCH + NPLRS)}{Mu\ VBP} \qquad \text{Equation 1}$$

Where:

NPCH is the volume of the channel,
NLRS is the volume of the LRS chamber,
Mu is the concentration of platelets at saturation, and
VBP is the confirmed volume of blood processed.

If the PCT determined in this manner is greater than a pre-determined upper limit (step 232), for example, 600 platelets per milliliter, an error condition is declared and the collection procedure is disqualified (step 236). Otherwise, the apparatus uses the newly-determined PCT to update the prediction model used by the apparatus to predict the product that can be collected and the estimated time for the collection procedure (step 234). The apparatus collects blood components according to its defined procedure. When completed, the apparatus begin rinseback procedures (step 238) to return remaining blood components to the donor.

In addition to the PCT (platelet pre-count), the above-described technique can be used to measure the hematocrit of the donor.

$$HCT = k* \frac{(NRCH + NRLRS)}{Mu\ VBP} \qquad \text{Equation 2}$$

Where:

HCT is the hematocrit,
NRCH is the volume of the channel,
NRLRS is the volume of the LRS chamber,
Mu is the concentration of red blood cells resulting from separation in the apparatus,
VBP is the confirmed volume of blood processed, and
k is a constant dependant on the type of blood processing device (brand or configuration) and on the disposable vessel being used).

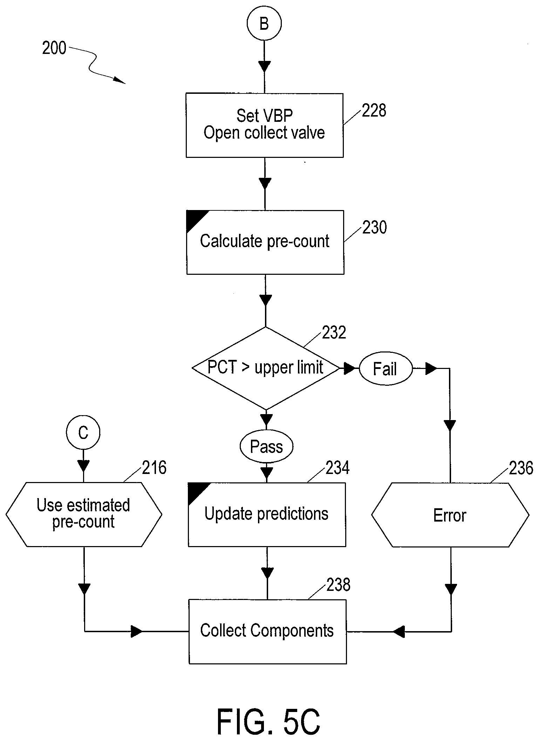

Because red blood cells are relatively opaque, they are readily detected by the RBC detector, which is designed for that purpose. The detection algorithm described in connection with Fig. 5A, 5B and 5C is not necessary. The constant k can be experimentally determined for any selected combination of blood processing device and disposable vessel by processing samples of blood of known hematocrit. The value of the constant k is expected to be between about 0.8 and 1.2.

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, are within the scope of the present invention as defined by the claims. The embodiments described hereinabove are further intended to explain best modes known of practicing the invention and to enable others skilled in the art to utilize the invention in such or other embodiments and with various modifications required by the particular application(s) or use(s) of the present invention as defined by the claims.

## Claims

1. A centrifugal blood processing apparatus (2) comprising:

a centrifuge rotor (20);
a blood processing vessel (12) for receiving whole blood and separating said whole blood into components;
a tubing set (8) adapted to be mounted on said rotor (20) for conducting blood components and fluids, said tubing set (8) having a leukoreduction chamber (53) for periodically accumulating platelets received from said blood processing vessel (12), said leukoreduction chamber (53) being configured to form a fluidized bed of platelets in the leukoreduction chamber (53);
a light-detecting sensor (74), said sensor (74) being adapted to be placed in optical communication with at least part of said tubing set (8);
means in electrical communication with said sensor (74) for detecting spill-over of platelets out of said leukoreduction chamber (53), and
means for determining said platelet pre-count from at least a known volume associated with said leukoreduction chamber (53) and a measured volume of blood that had entered said centrifugal blood processing apparatus (2) before said spill-over.

2. The apparatus of claim 1, wherein the means for determining said platelet pre-count comprises means for detecting spill-over of platelets by detecting average reflectance of fluid flowing out of said leukoreduction chamber (53) and detecting when a current value for said average reflectance is less than a pre-determined part of an initial value for said average reflectance.

3. The apparatus of claim 1, wherein the means for determining said platelet pre-count comprises means for detecting spill-over of platelets by determining if each sum of a series of sums of measurements of average reflectance for selected periods of time are less than zero, thereby indicating a relatively continuous decrease in reflectance during each of said selected periods of time.

4. The apparatus of claim 3, wherein the means for detecting spill-over of platelets by determining each sum of a series of sums further comprises means for determining if said sums for selected periods immediately prior to a current period are less than a predetermined upper limit, thereby indicating a relatively rapid change in reflectance.

5. The apparatus of claim 4, wherein the means for detecting spill-over of platelets by determining each sum of a series of sums further comprises means for combining all sums for a selected period of time immediately preceding a current time and determining that said combined sum is less than a pre-determined value.

6. The apparatus of claim 5, wherein the means for detecting spill-over of platelets by determining each sum of a series of sums further comprises means for determining if the differences between each two adjacent sums during a pre-selected period of time is less than zero.

## Patentansprüche

1. Zentrifugale Blutverarbeitungsvorrichtung (2), umfassend:

einen Zentrifugenrotor (20);
ein Blutbehandlungsgefäß (12) zum Aufnehmen von Vollblut und Auftrennen des Vollbluts in Komponenten;
ein Schlauchset (8), das angepasst ist, um an dem Rotor (20) zum Leiten von Blutkomponenten und Flüssigkeiten montiert zu sein, wobei das Schlauchset (8) eine Leukoreduktionskammer (53) zum periodischen Akkumulieren von Blutplättchen hat, die von dem Blutbehandlungsgefäß (12) aufgenommen werden, wobei die Leukoreduktionskammer (53) konfiguriert ist, um eine Wirbelschicht aus Blutplättchen in der Leukoreduktionskammer (53) zu bilden;
einen Lichterfassungssensor (74), wobei der Sensor (74) angepasst ist, um in optische Verbindung mit mindestens einem Teil des Schlauchsets (8) gesetzt zu sein;
ein Mittel in elektrischer Verbindung mit dem Sensor (74) zum Erfassen von einem Überlaufen von Blutplättchen aus der Leukoreduktionskammer (53) heraus und
ein Mittel zum Bestimmen der Blutplättchen-Vorzählung von mindestens einer bekannten Menge in Zusammenhang mit der Leukoreduktionskammer (53) und einer gemessenen Menge von Blut, die die zentrifugale Blutverarbeitungsvorrichtung (2) vor dem Überlaufen betreten hat.

2. Vorrichtung nach Anspruch 1, wobei das Mittel zum Bestimmen der Blutplättchen-Vorzählung ein Mittel zum Erfassen von einem Überlaufen von Blutplättchen durch das Erfassen von einem durchschnittlichen Reflexionsgrad von Flüssigkeit, die aus der Leukoreduktionskammer (53) fließt, und das Erfassen umfasst, wenn ein aktueller Wert des durchschnittlichen Reflexionsgrads weniger als ein vorbestimmter Teil eines Anfangswerts für den durchschnittlichen Reflexionsgrad ist.

3. Vorrichtung nach Anspruch 1, wobei das Mittel zum Bestimmen der Blutplättchen-Vorzählung ein Mittel zum Erfassen von einem Überlaufen von Blutplättchen durch das Bestimmen umfasst, ob jede Summe einer Reihe von Summen von Messungen von durchschnittlichem Reflexionsgrad für ausgewählte Zeiträume weniger als null sind, wodurch eine relativ kontinuierliche Abnahme an dem Reflexionsgrad während jedem der ausgewählten Zeiträume angezeigt wird.

4. Vorrichtung nach Anspruch 3, wobei das Mittel zum Erfassen von einem Überlaufen von Blutplättchen durch Bestimmen jeder Summe einer Reihe von Summen ferner ein Mittel zum Bestimmen umfasst, ob die Summen für ausgewählte Zeiträume unmittelbar vor einem aktuellen Zeitraum weniger als ein vorbestimmtes oberes Limit sind, wodurch eine relativ schnelle Veränderung des Reflexionsgrads angezeigt wird.

5. Vorrichtung nach Anspruch 4, wobei das Mittel zum Erfassen von einem Überlaufen von Blutplättchen durch Bestimmen jeder Summe einer Reihe von Summen ferner ein Mittel zum Kombinieren von allen Summen für einen ausgewählten Zeitraum, der einer aktuellen Zeit unmittelbar vorhergeht, und zum Bestimmen umfasst, dass die kombinierte Summe weniger als ein vorbestimmter Wert ist.

6. Vorrichtung nach Anspruch 5, wobei das Mittel zum Erfassen von einem Überlaufen von Blutplättchen durch Bestimmen jeder Summe einer Reihe von Summen ferner ein Mittel zum Bestimmen umfasst, ob die Unterschiede zwischen jeweils zwei benachbarten Summen während einem vorgewählten Zeitraum weniger als null ist.

## Revendications

1. Appareil de traitement du sang par centrifugation (2) comprenant :

   un rotor centrifuge (20) ;
   un récipient de traitement du sang (12) destiné à recevoir du sang total et séparer ledit sang total en composants ;
   un jeu de tubes (8) conçu pour être monté sur ledit rotor (20) destiné à conduire des composants sanguins et fluides, ledit jeu de tubes (8) comportant une chambre de leucoréduction (53) permettant d'accumuler périodiquement des plaquettes provenant dudit récipient de traitement du sang (12), ladite chambre de leucoréduction (53) étant conçue pour former un lit fluidisé de plaquettes dans la chambre de leucoréduction (53) ;
   un capteur de détection de lumière (74), ledit capteur (74) étant conçu pour être placé en communication optique avec au moins une partie dudit jeu de tubes (8) ;
   un moyen en communication électrique avec ledit capteur (74) destiné à détecter un débordement de plaquettes hors de ladite chambre de leucoréduction (53), et
   un moyen de détermination de la numération plaquettaire préalable à partir d'au moins un volume connu associé à ladite chambre de leucoréduction (53) et un volume mesuré de sang qui était entré dans ledit appareil de traitement du sang par centrifugation (2) avant ledit débordement.

2. Appareil selon la revendication 1, dans lequel le moyen de détermination de ladite numération plaquettaire préalable comprend un moyen de détection d'un débordement de plaquettes par la détection d'un facteur de réflexion moyen d'un fluide s'écoulant hors de ladite chambre de leucoréduction (53) et la détection du moment où une valeur actuelle pour ledit facteur de réflexion moyen est inférieure à une partie prédéterminée d'une valeur initiale pour ledit facteur de réflexion moyen.

3. Appareil selon la revendication 1, dans lequel le moyen de détermination de ladite numération plaquettaire préalable comprend un moyen de détection d'un débordement de plaquettes par la détermination de savoir si chaque somme d'une série de sommes de mesures d'un facteur de réflexion moyen pour des périodes sélectionnées de temps est inférieure à zéro, permettant ainsi d'indiquer une diminution relativement continue du facteur de réflexion pendant chacune desdites périodes de temps sélectionnées.

**EP 2 788 043 B1**

4. Appareil selon la revendication 3, dans lequel le moyen de détection d'un débordement de plaquettes par la détermination de chaque somme d'une série de sommes comprend en outre un moyen de détermination de savoir si lesdites sommes pour des périodes sélectionnées immédiatement avant une période actuelle sont inférieures à une limite supérieure prédéterminée, permettant ainsi d'indiquer un changement relativement rapide du facteur de réflexion.

5. Appareil selon la revendication 4, dans lequel le moyen de détection d'un débordement de plaquettes par la détermination de chaque somme d'une série de sommes comprend en outre le moyen de combinaison de toutes les sommes pour une période de temps sélectionnée précédant immédiatement un moment actuel et de détermination que ladite somme combinée est inférieure à une valeur prédéterminée.

6. Appareil selon la revendication 5, dans lequel le moyen de détection d'un débordement de plaquettes par la détermination que chaque somme d'une série de sommes comprend en outre un moyen de détermination de savoir si les différences entre chacune des deux sommes adjacentes pendant une période de temps présélectionnée est inférieure à zéro.

FIG. 1

FIG. 2

74

132

130

SIGNAL
COMPARATOR

126

128

120

RED EMIT/DETECT
STATION

124

GREEN EMIT/DETECT
STATION

52

122

## FIG. 3

74

134

RED EMIT/DETECT
STATION

122

52

138

136

GREEN EMIT/DETECT
STATION

SIGNAL
COMPARATOR

132

130

140

## FIG. 4

200

| Initiate | 202 |

| Prime | 204 |

| Monitor
RBC detector | 206 |

| Record &
average | 208 |

| Calculate &
store differences | 210 |

| Sum last differences | 212 |

A

# FIG. 5A

FIG. 5B

200

B

Set VBP
Open collect valve — 228

Calculate pre-count — 230

PCT > upper limit — 232

Fail

C

Use estimated
pre-count — 216

Pass

Update predictions — 234

Error — 236

Collect Components — 238

FIG. 5C

## REFERENCES CITED IN THE DESCRIPTION

### Patent documents cited in the description

- US 7052606 B
- US 6773413 B
- US 6200287 B
- US 12234960 B
- US 5734464 A
- US 20090211989 A
- US 5734494 A
- US 12423883 B
- US 12234940 B